# EUROPEAN PATENT SPECIFICATION

(11) **EP 2 205 279 B1**
(45) Date of publication and mention of the grant of the patent: **13.04.2011**
(21) Application number: 08835893.2
(22) Date of filing: 24.09.2008
(51) Int. Cl.: A61K 45/06

(54) **PHARMACEUTICAL COMBINATION OF ALISKIREN AND VALSARTAN**
PHARMAZEUTISCHE KOMBINATION AUS ALISKIREN UND VALSARTAN
COMBINAISON PHARMACEUTIQUE D'ALISKIRÈNE ET DE VALSARTANE

(30) Priority: 28.09.2007 US 975905 P
(43) Date of publication of application: 14.07.2010
(73) Proprietor: Novartis AG, 4056 Basel (CH)
(72) Inventor: CURDY, Catherine, CH-4125 Riehen (CH); LI, Shoufeng, Basking Ridge New Jersey 07920 (US); BECKER, Dieter, 79102 Freiburg (DE); SCHLÜTERMANN, Burkhard, 79280 Au (DE); GERBER, Frédéric, F-68730 Blotzheim (FR)
(74) Representative: Graff, Alan
(86) International application number: PCT/US2008/077417
(87) International publication number: WO 2009/045796

(56) References cited:
- WO-A-2006/102177
- WO-A-2007/001066
- US-A1- 2006 018 960

## Description

The present invention relates to pharmaceutical oral fixed dose combinations comprising an orally active renin inhibitor, Aliskiren, or a pharmaceutically acceptable salt thereof, and an angiotensin II antagonist, Valsartan, or a pharmaceutically acceptable salt thereof, as the active ingredients in a suitable carrier. In particular, the present invention provides galenical formulations comprising the hemi-fumarate salt of Aliskiren in combination with Valsartan. The present invention also relates to the processes for their preparation and to their use as medicaments.

Renin released from the kidneys cleaves angiotensinogen in the circulation to form the decapeptide angiotensin I. This is in turn cleaved by angiotensin converting enzyme in the lungs, kidneys and other organs to form the octapeptide angiotensin II. The octapeptide increases blood pressure both directly by arterial vasoconstriction and indirectly by liberating from the adrenal glands the sodium-ion-retaining hormone aldosterone, accompanied by an increase in extracellular fluid volume. Inhibitors of the enzymatic activity of renin bring about a reduction in the formation of angiotensin I. As a result a smaller amount of angiotensin II is produced. The reduced concentration of that active peptide hormone is the direct cause of, e.g., the antihypertensive effect of renin inhibitors. Accordingly, renin inhibitors, or salts thereof, may be employed, e.g., as antihypertensives or for treating congestive heart failure.

The renin inhibitor, Aliskiren, in particular, a hemi-fumarate thereof, is known to be effective in the treatment of reducing blood pressure irrespective of age, sex or race and is also well tolerated. Aliskiren in form of the free base is represented by the following formula 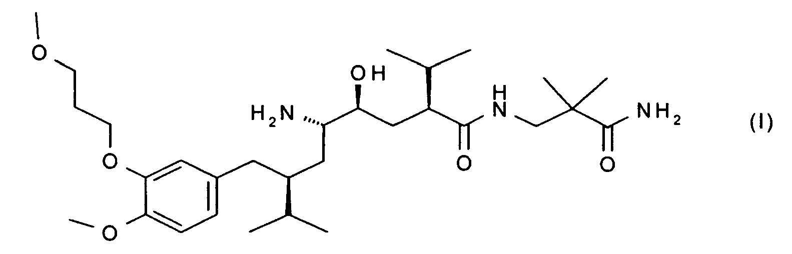 and chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide. As described above, most preferred is the hemi-fumarate salt thereof which is specifically disclosed in EP 678503 A as Example 83.

Valsartan is a known Angiotensin receptor blocker (ARB, angiotensin II antagonist) and the combination with Aliskiren is described, e.g. in WO02/40007.

Angiotensin II is a hormone that causes blood vessels to constrict. This, in turn, can result in high blood pressure and strain on the heart. It is known that angiotensin II interacts with specific receptors on the surface of target cells. Two receptor subtypes for angiotensin II, namely AT1 and AT2, have been identified thus far. In recent times, great efforts have been made to identify substances that bind to the AT1 receptor. Angiotensin receptor blockers (ARBs, angiotensin II antagonists) are now known to prevent angiotensin II from binding to its receptors in the walls of blood vessels, thereby resulting in lower blood pressure. Because of the inhibition of the AT1 receptor, such antagonists can be used, therefore, as anti-hypertensives or for the treatment of congestive heart failure, among other indications.

Administration of such pharmaceutical agents via the oral route is preferred to parenteral administration because it allow self-administration by patients whereas parenteral formulations have to be administered in most cases by a physician or paramedical personnel.

However, Aliskiren is a drug substance difficult to formulate due to its physicochemical properties and it is not trivial to make oral formulations in the form of tablets in a reliable and robust way. For example, Aliskiren has a needle shaped crystallization habit, which has a negative influence on the bulk properties of the drug substance, e.g., flow properties and bulk density. The compression behavior of the drug substance is poor, leading to weak interparticulate bonds and polymorphism changes under pressure. Aliskiren has a strong elastic component that also leads to weakening of interparticulate bonds. The drug substance quality is very variable with effect on the processability of a tablet, e.g., particle size distribution, bulk density, flowability, wetting behavior, surface area and sticking tendency. Moreover, Aliskiren is highly hygroscopic. After contact with water and removal of the water, the drug substance polymorphism changes to an amorphous state, which shows inferior stability compared to the crystalline state. In addition, in the particular case of high dose of Aliskiren or a pharmaceutically acceptable salt thereof (up to 300 mg of the free base per tablet) makes a high drug loading necessary in order to achieve a reasonable tablet size.

The combination of these hurdles makes a standard tablet manufacturing process extremely difficult. A solid oral dosage form of Aliskiren is described in W02005/089729.

On the other hand, Valsartan has pH dependent solubility whereby it ranges from very slightly soluble in an acidic environment to soluble in a neutral environment of the gastrointestinal tract. Further, development of a patient-convenient oral dosage form of Valsartan is challenging due to its low bulk density.

Moreover, in general the development of oral fixed dose combination formulations using certain active ingredients is challenging. As used herein, "fixed dose combination" refers to a combination of defined doses of two drugs or active ingredients presented in a single dosage unit (e.g. a tablet or a capsule) and administered as such; further as used herein, "free dose combination" refers to a combination of two drugs or active ingredients administered simultaneously but as two distinct dosage units. When formulating oral fixed dose combinations, it is of advantage to provide a patient-convenient dosage form that is bioequivalent to the corresponding free dose combination of the same active ingredients in order to save time and costs in the development of the fixed dose combination. Development of fixed-dose combinations that are bioequivalent to the free dose combination is challenging due to the multiplicity of hurdles arising from pharmacokinetic and pharmaceutical properties of the drugs sought to be combined.

The difficulties encountered with Aliskiren to prepare oral formulations in the form of tablets in a reliable and robust way are believed to be potentiated when using it in combination with other therapeutic agents, in particular Valsartan for the reasons mentioned above.

In the case where the therapeutic doses of Valsartan and Aliskiren are high, when the two drugs are combined it is highly desired that the amounts of excipients are kept at a minimum to avoid excessively large formulations. Despite that fact, the formulation should still fulfill all of the above requirements.

Accordingly, a suitable and robust galenical formulation overcoming the above problems related to the properties of Aliskiren in particular when formulated together with Valsartan need to be developed.

Surprisingly it has been found that a certain dissolution profile of the two active ingredients is required in order to achieve a robust galenical formulation of the combination which is as similar as possible to the corresponding free dose combination with regard to the area under the curve (AUC) and preferably also the maximum plasma concentration (Cmax) so as to be most preferably bioequivalent to the free combination of the two active ingredients. From the solubility and absorption properties of the individual active ingredients one skilled in the art would not expect that the dissolution profile is critical in approaching or reaching bioequivalence.

In one embodiment, the present invention is directed to a pharmaceutical oral fixed dose combination comprising
a) a therapeutically effective amount of Aliskiren, or a pharmaceutically acceptable salt thereof,
b) a therapeutically effective amount of Valsartan, or a pharmaceutically acceptable salt thereof,
wherein the pharmaceutical oral fixed dose combination shows an in vitro dissolution of component (a) of 60% or less, such as of from 60% to 15%, after 10 minutes and 95% or less, such as of from 95% to 40%, after 20 minutes, and a dissolution profile of component (b) of 25 % or more, such as of 30% or more, after 30 minutes, and 45% or more, such as 60% or more after 60 minutes at pH 4.5.

Such a pharmaceutical oral fixed dose combination has an AUC and preferably also a Cmax for the respective active ingredients which is as similar as possible to the to a free dose combination of Aliskiren and Valsartan and such a pharmaceutical oral fixed dose combination is most preferably bioequivalent to such a free combination. It was surprising that the above dissolution data were so crucial since for Aliskiren and Valsartan it should not matter at which rate the active ingredient is released during the first 20 min and 60 min, respectively. As a BCS (biopharmaceutical classification system) class 3 compound (high solubility, low permeability), the release rate and subsequent dissolution rate for Aliskiren from the fixed dose combination should not be critical as long as the dissolution rate is similar or faster than for the existing Aliskiren film-coated tablets. Indeed, one of the pharmacokinetic parameters, the area under the curve (AUC) is taken over a period of 24 h so that the release rate and subsequent dissolution rate during the first 1h or less should not be that important. Nevertheless, it was found that if the dissolution profile for at least one of the components, i.e. Aliskiren or Valsartan, typically the dissolution profile for Aliskiren, was outside the above-mentioned ranges, no similarity in AUC and/or Cmax and thus no bioequivalence for the fixed dose combination was found in human pharmacokinetic studies. For example in the case of Aliskiren, a faster dissolution than as mentioned above leads to a substantially lower exposure from the fixed combination compared to that from the free combination. It is surprising to find that an inverse relationship exists for Aliskiren between dissolution and absorption, whereby a dosage form with a faster dissolution of Aliskiren has lower bioavailability.

Throughout the present application, the various terms are as defined below:

### Release profile:

The term "release" as used herein refers to a process by which the pharmaceutical oral fixed dose combination is brought into contact with a fluid and the fluid transports the drug(s) outside the dosage form into the fluid that surrounds the dosage form. The combination of delivery rate and delivery duration exhibited by a given dosage form in a patient can be described as its in vivo release profile. The release profiles of dosage forms may exhibit different rates and durations of release and may be continuous. Continuous release profiles include release profiles in which one or more active ingredients are released continuously, either at a constant or variable rate.

When two or more components that have different release profiles are combined in one dosage form, the resulting individual release profiles of the two components may be the same or different compared to a dosage form having only one of the components. Thus, the two components can affect each other's release profile leading to a different release profile for each individual component.

A two-component dosage form can exhibit release profiles of the two components that are identical or different to each other. The release profile of a two-component dosage form where each component has a different release profile may be described as "asynchronous". Such a release profile encompasses both (1) different continuous releases where preferably component b) is released at a slower rate than component a), and (2) a profile where one of components a) and b), preferably component b), is released continuous and the other of components a) and b), preferably component a), is modified to be released continuous with a time delay. Also a combination of two release profiles for one drug is possible e.g. 50% of the drug in continuous and 50% of the same drug continuous with a time delay.

### Immediate release:

For the purposes of the present application, an immediate release formulation is a formulation showing a release of the active substance(s), which is not deliberately modified by a special formulation design or manufacturing method.

### Modified release:

For the purposes of the present application, a modified release formulation is a formulation showing a release of the active substance(s), which is deliberately modified by a special formulation design or manufacturing method. This modified release can be typically obtained by delaying the time of release of one or both of the components, preferably component a). Typically for the purposes of the present invention, a modified release refers to a release over 5 h, such as a release over 3 h or even shorter. Modified release as used herein is meant to encompass both a different continuous release over time of the two components or a delayed release where one of the components, preferably component a), is released only after a lag time. Such a modified release form may be produced by applying release-modifying coatings, e.g. a diffusion coating, to the drug substance(s) or to a core containing the drug substance(s), or by creating a release-modifying matrix embedding the drug substance(s).

The term "time delay" as used herein refers to the period of time between the administration of a dosage form comprising the composition of the invention and the release of the active ingredient from a particular component thereof.

The term "lag time" as used herein refers to the time between the release of the active ingredient from one component of the dosage form and the release of the active ingredient from another component of the dosage form.

### Disintegration:

The term "disintegration" as used herein refers to a process where the pharmaceutical oral fixed dose combination, typically by means of a fluid, falls apart into separate particles and is dispersed. Disintegration is achieved when the solid oral dosage form is in a state in which any residue of the solid oral dosage form, except fragments of insoluble coating or capsule shell, if present, remaining on the screen of the test apparatus is a soft mass having no palpably firm core in accordance with USP<701 >. The fluid for determining the disintegration property is water, such as tap water or deionized water. The disintegration time is measured by standard methods known to the person skilled in the art, see the harmonized procedure set forth in the pharmacopeias USP <701> and EP 2.9.1 and JP.

### Erosion:

The term "erosion" as used herein refers to a process by which the pharmaceutical oral fixed dose combination may be worn away, diminished or dissolved when placed in an external environment (e.g. dissolution medium, body fluids etc.). In contrast to disintegration, the pharmaceutical oral fixed dose combination is not dispersed by falling apart, rather it is becoming smaller with time as the erosion process proceeds.

### Dissolution rate:

The term "dissolution" as used herein refers to a process by which a solid substance, here the active ingredients, is dispersed in molecular form in a medium. The dissolution rate of the active ingredients of the pharmaceutical oral fixed dose combination of the invention is defined by the amount of drug substance that goes in solution per unit time under standardized conditions of liquid/solid interface, temperature and solvent composition. The dissolution rate is measured by standard methods known to the person skilled in the art, see the harmonized procedure set forth in the pharmacopeias USP <711 > and EP 2.9.3 and JP. For the purposes of this invention, the test is for measuring the dissolution of the individual active ingredients is performed following pharmacopeia USP <711> at pH 4.5 using a paddle stirring element at 75 rpm (rotations per minute). The dissolution medium is preferably a buffer, typically a phosphate buffer, especially one as described in the example "Dissolution Test". The molarity of the buffer is preferably 0.1 M.

### Physically separated:

The term "physically separated" as defined herein refers to a pharmaceutical oral fixed dose combination containing both components a) and b) formulated to minimize physical contact such that the dissolution profile is as similar as possible to the free dose combination of a) and b) with regard to the area under the curve (AUC) and preferably also the maximum plasma concentration (Cmax) so as to approach or reach bioequivalence. In one embodiment, "physically separated" refers to a pharmaceutical oral fixed dose combination containing both components a) and b) formulated such that they are not mixed with each other in the same carrier but are separated. This separation helps to minimize the interactions between the two components especially upon release of same. Typically the physical separation means that the two components a) and b) are present in different compartments, such as layers, or are present as different entities, such as particulates or granulates, of the formulation. It is not necessary that he two components a) and b) are further separated by additional layers or coating although this may be appropriate from case to case. This physical separation of the two components a) and b) in one dosage form can be achieved by various means known in the art.

In one embodiment, this is achieved by formulating the respective components a) and b) into separate layers, coats or shells, preferably layers or shells to obtain, e.g. a multi- or bilayer formulation, a dry-coated (core in a shell) tablet, a molded delivery system, or a spray coated tablet, preferably to obtain a bilayer formulation or a dry-coated formulation. Specific examples of such formulation techniques are described hereinafter.

In another embodiment, this is achieved by using particulate systems (multiparticulates) that comprise particles of different populations of component a) and component b), respectively, to obtain, e.g. capsules, sachets, stickpacks filled with multiparticulates, tablets obtained from compressing multiparticulates, and minitablets obtained from compressing multiparticulates, such as granules or beads, which can subsequently be filled into capsules. Another form of a physical separation is a capsule filled with 1) multiparticulates of one of the components and 2) one tablet, several tablets or minitablets obtained from compressing multiparticulates, such as granules or beads, of the other component.

One can also consider any combination of the above two approaches such as multiparticulates, such as pellets, or minitablets provided with a layer, coat or shell where the layer, coat or shell contains one of the components a) and b) and the multiparticulates or minitablets contain the other of the components a) and b).

The term "particulate" as used herein refers to a state of matter which is characterized by the presence of discrete particles, pellets, beads or granules irrespective of their size, shape or morphology. When a plurality of particulates is present, these are referred to a multiparticulates. Typically, the particulates have an average size of lower than of from 3 mm, preferably between 1 µm to 3 mm. By " average particle size" it is meant that at least 50% of the particulates have a particle size of less than about the given value, by weight. The particle size may be determined on the basis of the weight average particle size as measured by conventional particle size measuring techniques well known to those skilled in the art. Such techniques include, for example, sedimentation field flow fractionation, photon correlation spectroscopy, light scattering, and disk centrifugation. If a mixture of multiparticulates component a) and component b) are used, the multiparticulates of component a) and b) may be in the same form (e.g. granules) and/or size or the multiparticulate sytem for one of the components may be in one form (e.g. particles) and size and e multiparticulate sytem for the other component may be in a different form (e.g. granules) and/or size.

The term "small tablets" within the scope of this application denotes tablets with an overall size of from 3 to 5 mm.

The term "minitablets" within the scope of this application denotes small tablets with an overall weight of approximately 2 to 30 mg, e.g. approximately 4 to 9 mg, e.g. approximately 7 mg, in their uncoated form. Minitablets are a specific form of multiparticulates as defined herein. They can be prepared as described herein, including preparation from other, smaller multiparticulates, such as granules or beads. The minitablets may have any shape known to the skilled person for tablets, e.g. round e.g. with a diameter of from 1.25 to 3 mm; cyclindrical e.g. having a convex upper face and convex lower face and e.g. with a cylindrical diameter and height independently of each other are from 1 to 3 mm; or biconvex minitablets e.g. whose height and diameter are approximately equal and are from 1.25 to 3 mm. Preferably, multiparticulates have a modified release coating. Specifically, if a mixture of multiparticulates component a) and component b) are used, the respective multiparticulates comprise different modified release coatings in order to provide different modified release profiles especially for component a).

### Bioequivalence:

The term "bioequivalence" as used herein is related to bioavailability as follows. The term "bioavailability", as used herein, is defined as a measure of the rate and amount of active ingredient which reaches the systemic circulation unchanged following the administration of the dosage form. The bioavailability of pharmaceutical oral fixed dose combination of the present invention is compared with that of the corresponding free dose combinations. The test (fixed dose combination) and the reference (free dose combination) formulations are administered orally to the subjects, and plasma samples are collected over time. The plasma samples are analyzed for concentration of Valsartan and Aliskiren. The maximum plasma concentration (Cmax) and the area under the plasma concentration vs. time curve (AUC) are calculated. Log-transformed AUC0-tlast (AUC from time zero to the last measurable concentration sampling time), AUC0-∞ (AUC from time zero to infinity), Cmax of aliskiren and valsartan are analyzed separately using a linear mixed effects model, with fixed effects from sequence, treatment and period, and random effects from subject. A point estimate (ratio of geometric mean of Cmax or AUC for test versus reference formulation) and the corresponding 90% confidence intervals are used to evaluate bioequivalence. For the test and reference products to be bioequivalent, the 90% confidence intervals for both AUC and Cmax point estimates should fall within 0.8-1.25. Obtaining bioequivalence between test and reference products is challenging, particularly for combinations of active ingredients, and the result cannot be predicted a priori.

Whenever reference is made to an AUC being similar to the active ingredient in the free combination, it is meant that the AUC in the pharmaceutical oral fixed dose combination of the present invention has preferably a 90% confidence interval which should fall within 0.8-1.25 for the active ingredients.

Whenever reference is made to an Cmax being similar to the active ingredient in the free combination, it is meant that the Cmax in the pharmaceutical oral fixed dose combination of the present invention has preferably a 90% confidence interval which should fall within 0.8-1.25 for the active ingredients.

In a preferred embodiment, the pharmaceutical oral fixed dose combination of the present invention has a release profile for one or both of the active ingredients, in particular for Aliskiren, such that the AUC and Cmax point estimate(s) are in the range of from 0.8 to 1.3, more preferably of from 0.8 to 1.25, most preferably of from 0.85 to 1.2.

In another embodiment, the pharmaceutical oral fixed dose combination of the present invention has a release profile for one or both of the active ingredients, in particular for Aliskiren, such that the 90% confidence interval for AUC(s) and Cmax are, of from 0.7 to 1.43, more preferably of from 0.7 to 1.30, still more preferably of from 0.75 to 1.25, most preferably of from 0.8 to 1.25,

In another embodiment, the pharmaceutical oral fixed dose combination of the present invention has a release profile for one or both of the active ingredients, in particular for Aliskiren, such that the 90% confidence interval for AUC(s) and Cmax are, of from 0.7 to 1.43, more preferably of from 0.7 to 1.30, still more preferably of from 0.75 to 1.25, most preferably of from 0.8 to 1.25,

It is preferred that at least the AUC(s), more preferably both the AUC(s) and the Cmax(s) are within the above-mentioned ranges.

By virtue of this the pharmaceutical oral fixed dose combination of the present invention will approach or preferably reach bioequivalence.

In a preferred embodiment of the present invention, component (a) is present in an amount ranging from 10 to 45, such as 25 to 35%, by weight based on the total weight of the pharmaceutical oral fixed dose combination.

In another preferred embodiment of the present invention component (a) is present in an amount of 12 to 45, such as 12 to 40, in one embodiment 12 to 35, such as 12 to 25% by weight based on the total weight of the pharmaceutical oral fixed dose combination.

It is preferred that component (a) is present in an amount ranging of from 37.5 mg to 300 mg of the free base per unit pharmaceutical oral fixed dose combination.

In a preferred embodiment of the present invention, component (a) is present in an amount ranging from 75 to 300mg, such as 75 to 150 mg, of the free base per unit pharmaceutical oral fixed dose combination, in particular 75, 150 or 300 mg, such as 150 or 300 mg.

In a preferred embodiment of the present invention, component (b) is present in an amount ranging from 8 to 45 %, such as 10 to 35 %, in particular 12 to 32 %, by weight based on the total weight of the pharmaceutical oral fixed dose combination.

In a preferred embodiment of the present invention, component (b) is present in an amount of 20 to 40, such as 20 to 30%, by weight based on the total weight of the pharmaceutical oral fixed dose combination.

It is preferred that component (b) is present in an amount ranging from 10 to 640mg, such as 20 to 320 mg, more preferably 40 mg to 320 mg, such as 180 to 320 mg, per unit dosage form, in particular 80, 160 or 320 mg, such as 160 or 320 mg.

The weight ratio of component (a) to component (b) preferably ranges of from 1:0.001 to 1:5, more preferably of from 1:0.5 to 1:4 or 1:0.03 to 1:0.07. Most preferably, the weight ratio is of from 1:1.0 to1.1; 1:2.1 to 2.2; or 1:0.005 to 0.006 based on the free acids of (a) and (b). Most preferably, components (a) and (b), are used in amounts of 75/80 mg, 75/160 mg, 150/80 mg, 150/160 mg, 300/320 mg, 300/160 mg or 150/320 mg, most preferably 150/160 mg, 300/320 mg, 300/160 mg or 150/320 mg of (a)/(b), based on the free base of (a) and the free acid of (b). In one embodiment it is preferred to use a high drug load using 300 mg of (a) and/or 320 mg of (b), most preferably 300/320 mg of (a) and (b).

When using a salt such as the hemifumarate for component (a), the ratios will be adapted accordingly. For the following ratios, the numbers refer to component (a), thus referring to the free base or the salt, in particular the hemifumarate.

The terms "effective amount" or "therapeutically effective amount" refers to the amount of the active ingredient or agent which halts or reduces the progress of the condition being treated or which otherwise completely or partly cures or acts palliatively on the condition. The terms "drugs", "active substances", active ingredients", "active agents" etc. as used herein refer to components a) and b) unless specified otherwise. Each of component a) or b) can be referred to as a "drug", "active substance", active ingredient", "active agent" etc..

In the above and in the following the term "Aliskiren", if not defined specifically, is to be understood both as the free base and as a salt thereof, especially a pharmaceutically acceptable salt thereof, such as a hemi-fumarate, hydrogen sulfate, orotate or nitrate, most preferably a hemi-fumarate thereof.

Aliskiren, or a pharmaceutically acceptable salt thereof, can, e.g., be prepared in a manner known per se, especially as described in EP 678503 A, e.g. in Example 83.

In the following the term "Valsartan", if not defined specifically, is to be understood both as the free acid and as a salt thereof, especially a pharmaceutically acceptable salt thereof, as described below.

Valsartan , or a pharmaceutically acceptable salt thereof, can, e.g., be prepared in a manner known per se. Preferred salts forms include acid addition salts. The compounds having at least one acid group (e.g., COOH or 5-tetrazolyl) can also form salts with bases. Suitable salts with bases are, e.g., metal salts, such as alkali metal or alkaline earth metal salts, e.g., sodium, potassium, calcium or magnesium salts, or salts with ammonia or an organic amine, such as morpholine, thiomorpholine, piperidine, pyrrolidine, a mono-, di- or tri-lower alkylamine, e.g., ethyl-, tert-butyl-, diethyl-, diisopropyl-, triethyl-, tributyl- or dimethylpropylamine, or a mono-, di- or trihydroxy lower alkylamine, e.g., mono-, di- or triethanolamine. Corresponding internal salts may furthermore be formed. Salts which are unsuitable for pharmaceutical uses but which can be employed, e.g., for the isolation or purification of free compounds I or their pharmaceutically acceptable salts, are also included. Even more preferred salts are, e.g., selected from the mono-sodium salt in amorphous form; di-sodium salt of Valsartan in amorphous or crystalline form, especially in hydrate form, thereof. Mono-potassium salt of Valsartan in amorphous form; di-potassium salt of Valsartan in amorphous or crystalline form, especially in hydrate form, thereof.
Calcium salt of Valsartan in crystalline form, especially in hydrate form, primarily the tetrahydrate thereof; magnesium salt of Valsartan in crystalline form, especially in hydrate form, primarily the hexahydrate thereof; calcium/magnesium mixed salt of Valsartan in crystalline form, especially in hydrate form; bis-diethylammonium salt of Valsartan in crystalline form, especially in hydrate form; bis-dipropylammonium salt of Valsartan in crystalline form, especially in hydrate form; bis-dibutylammonium salt of Valsartan in crystalline form, especially in hydrate form, primarily the hemihydrate thereof; mono-L-arginine salt of Valsartan in amorphous form; bis-L-arginine salt of Valsartan in amorphous form; mono-L-lysine salt of Valsartan in amorphous form; bis-L-lysine salt of Valsartan in amorphous form.

Most preferably, Valsartan is used as the free acid.

The pharmaceutical oral fixed dose combination according to the present invention needs to be selected appropriately to show the desired dissolution profile. Typically, the pharmaceutical oral fixed dose combination is a solid dosage form.

The pharmaceutical oral fixed dose combination of the present invention preferably exhibits release profiles of both components a) and b), more preferably component a) that are regarded as modified release profiles. The pharmaceutical oral fixed dose combination of the present invention preferably exhibits a release profile of component b) that is regarded as an immediate release profile. In a preferred embodiment of the present invention, the release profiles of the two components of the pharmaceutical oral fixed dose combination are asynchronous. In one embodiment, both components are released continuously with an asynchronous release profile, whereby one of the components, preferably component a), is modified to be released at a slower continuous rate. In another embodiment, one of the components, preferably component a), is released with a time delay so as result in a time lag of component a) compared to component b).

Preferably, the pharmaceutical oral fixed dose combination of the present invention is designed in such a way that components a) and b) are physically separated. Typical technologies and formulation principles for pharmaceutical oral fixed dose combinations capable to match the required dissolution profile according to the present invention include multiparticulate systems.

Thus, the present invention is in particular related to a pharmaceutical oral fixed dose combination in the form of multiparticulate systems, by using multiparticulates as defined herein. Such multiparticulate systems typically comprise a mixture of multiparticulates, comprising particles of different populations of component a) and component b) respectively, which provide different release profiles for each drug containing particles, e.g. which comprise a modified release coating for component a) containing particles.

The multiparticulate units that sustain the release of component a may consist of uncoated matrix systems, coated cores or coated matrix systems. The matrix, coating or coated matrix system may provide a continuous or a discontinued release of the drug.

When the combination of the invention is in the form of a tablet or capsule, it is preferably a tablet or capsule which is able to disintegrate or dissolve to liberate, multiparticulates, comprising particles of different populations of component a) and component b), e.g. modified release coated multiparticles. The tablet or capsule may disintegrate or dissolve in the mouth, stomach or small intestine. The tablet or capsule may release the multiparticulates with modified release properties. Preferably the composition of the invention is in a modified release coated multiparticulate form for component a).

When the combination of the invention is in the form of minitablets, it is preferably filled into capsules or aluminium stickpacks, which may provide a high variability of administered doses with the same formulation. Minitablets are preferably also filled into capsules or either stickpacks or sachets.

Most preferably, the combination of the present invention is in the form of a tablet comprising particles of different populations of component a) and component b) respectively.

The composition with modified release according to the invention may conveniently be coated with a component which offers a sustained, continuous, gradual, or prolonged release of component a) and/or component b) in the body, preferably in the intestine, e.g. a modified release coating, e.g. a diffusion coating.

Examples of such modified release coating components are e.g. cellulose derivatives; e.g. ethylcellulose, e.g. Aquacoat® ECD, available from FMC; Surelease available from Colorcon, Ethylcellulose N grades, available from Dow, acrylic copolymers, preferably acrylic and methacrylic copolymers containing quaternary ammonium groups, e.g. tri(C₁₋₄alkyl)-ammonium methylmethacrylate groups, e.g. trimethylammonium methylmethacrylate groups, e.g. acrylic/ methacrylicacid-ester with different ratio of quarternary ammonium groups 20:1 RL/ 40:1 RS, e.g. such polymers commercially available from Röhm Pharma under the Trademarks, Eudragit RL^{R}, Eudragit RS^{R} or Eudragit NE^{R} or copolymers; and/or mixtures thereof. A ratio of about 75:25, preferably 90:10, preferably 95:5 by weight Eudragit RS^{R}:Eudragit RL^{R} is particularly preferred.

The modified release coating components may be in aqueous dispersion, e.g. as 30% aqueous dispersion, or organic solution, e.g. 12.5% organic solution. For example the modified release coating components is a mixture of Eudragit RL^{R} and Eudragit RS^{R} in 30% aqueous dispersion or 12.5% organic solution.

The amount of modified release coating components may be from about 30 to about 100 weight %, more preferably from about 50 to about 100 weight %, based on the total weight of the coating.

The film-forming polymer, e.g. diffusion coating, preferably comprises 5 to 95 weight %, more preferably 40-90 weight %, even more preferably 50-85 weight %, of the total weight of the film-coat composition.

The skilled person would adjust the nature and amount of modified release coating polymer to adjust as necessary the dissolution profile of components a) and/or b), contained in the composition of the invention.

The modified release coating may further include one or more further components or excipients, e.g. pore formers, a plasticizer, an antisticking agent, a wetting agent, e.g. as disclosed hereinafter.

Suitable pore-formers may be pH independent pore-formers, such as HPMC, HPC or pore-formers which are pH dependent, Suitable pH dependent pore-formers may be enteric pore-formers, e.g. enteric coating polymers. These enteric polymers can also be used as film-forming agent and are included in the above section on film-forming agents.

As herein defined, an enteric pore-former is a pore-former which provides drug release in an environment with pH > 5, e.g. in intestinal fluid, and suppresses drug release in acidic environment, e.g. in the stomach. Example of enteric pore-formers according to the present invention are HPMC-phthalate (HPMC-P), e.g. HP50, HP55, e.g. from ShinEtsu; HPMC-acetate-succinate (HPMC-AS), e.g. Aqoat LF or Aqoat MF, e.g. from ShinEtsu; Methyl acrylic acid-ethyl acrylic acid copolymer, e.g. Methacrylic acid copolymer, e.g. Eudragit L, S, L100-55 and/or L30D from Röhm Pharma, Acryl-Eze from Colorcon, Kollicoat MAE 30 DP from BASF; Celluloseacetatephthalate, e.g. Aquacoat CPD from FMC Biopolymer, or Polymer from Eastman Kodak; and Polyvinylacetatephthalate, e.g. Sureteric, Colorcon, or any mixture thereof. Preferably HPMC-P and HPMC-AS may be combined with ethylcellulose or acrylic and methacrylic copolymers containing quaternary ammonium groups, e.g. tri(C₁₋₄alkyl)-ammonium methylmethacrylate groups, e.g. Eudragit RS in organic coating solutions, HPMC-AS dispersed in water can also be combined with aqueous ethylcellulose dispersion e.g. Aquacoat ECD, FMC.

Hydroxypropyl methylcellulose phthalates, typically have a molecular weight of from 20,000 to 100,000 Daltons e.g. 80,000 to 130,000 Daltons, e.g. a hydroxypropyl content of from 5 to 10%, a methoxy content of from 18 to 24% and a phthalyl content from 21 to 35%. Examples of suitable hydroxypropyl methylcellulose phthalates are the marketed products having a hydroxypropyl content of from 6-10%, a methoxy content of from 20-24%, a phthalyl content of from 21-27%, a molecular weight of about 84,000 Daltons known under the trade mark HP50 and available from Shin-Etsu Chemical Co. Ltd., Tokyo, Japan, and having a hydroxypropyl content, a methoxy content, and a phthalyl content of 5-9%, 18-22% and 27-35% respectively, and a molecular weight of 78,000 Daltons, known under the trademark HP55 and available from the same supplier.

Examples of suitable hydroxypropylmethylcellulose acetate succinate may be used as known under the trademark Aqoat LF or Aqoat MF and commercially available, e.g. from Shin-Etsu Chemical Co. Ltd., Tokyo, Japan.

The composition of the invention may further include a pH independent pore-former, e.g. which gives water-soluble pores, e.g. polyethyleneglycol, polyvinylpyrrolidone, polyethylene oxide, a cellulose derivative, e.g. hydroxyethyl cellulose, Hydroxypropylmethylcellulose (HPMC), Hydroxypropylcellulose (HPC), or other cellulose derivatives, e.g. which are soluble in acidic medium, e.g. as ammonium salt, acrylate or methacrylate esters, e.g.Eudragit E or Eudragit EPO; polyacrylic acid; which are swelling in water, e.g. Eudragit RS, RL, NE 30D, which are soluble in alkaline medium, i.e. enteric coating polymer, e.g. Eudragit L, S, L100-55 or any mixture thereof. HPMC may also act as a thickening agent due to the viscosity of the aqueous solution thereof. According to the invention the pore formers may be hydrophilic agents, e.g. water soluble plasticizers, e.g. PEG, triacetine, triethylcitrate, or hydrophilic silicium dioxide, e.g. Aerosil 200 or Syloid 244 FP.

The modified release coating of the composition of the invention may comprise 0 to 70 weight %, more preferably 5 to 50 weight %, most preferably 5 to 25 weight % of pore-former, based on the total weight of the modified release coating.

Suitable plasticizers according to the invention include e.g., triacetine, triethy citrate, tributyl citrate, dibutylsebacate, diethyl sebacate, polyethyleneglycol 400, 3000, 4000 or 6000, acetyltriethylcitrate, acetyltributylcitrate and diethylphthalate, or mixtures thereof. A plasticizer generally swells the coating polymer such that the polymer's glass transition temperature is lowered, its flexibility and toughness increased and its permeability altered. When the plasticizer is hydrophilic, such as polyethylene glycol, the water permeability of the coating is generally increased. When the plasticizer is hydrophobic, such as diethyl phthalate or dibutyl sebacate, the water permeability of the coating is generally decreased.

The plasticizer is preferably present in an amount of 0 to 50% by weight, preferably 2 to 35%,more preferable 5-25% based on the total weight of the coating.

Examples of antisticking agents are silicon dioxide, e.g. colloidal silicon dioxide, an synthetic amorphous silicic acid such as Syloid 244 FP, talc, Aerosil 200 or glycerine monostearate. Preferably the antisticking agent is Areosil 200 and Syloid 244 FP. When the antisticking agent is hydrophilic, such as Aerosil 200 or Syloid 244 FP, the water permeability/swelling (and therefore also drug release) of the coating is generally increased. When the antisticking agent is hydrophobic, such as talcum or glycerolmonostearate, the water permeability of the coating is generally decreased. Antisticking agents are optionally included in the coating formulation to avoid sticking of the drug cores and guarantee a high separation of them.

Preferably the antisticking agent is present in an amount of 0 to 50% by weight, more preferably 5 to 25% by weight, based on the total weight of the coating.

Suitable wetting agents include e.g. sodium laurylsulphate, cetomacrogol, a wax, glycerol monostearate, a sorbitan ester and a poloxamer. Wetting agents are optionally included in the coating formulation due to their property to reduce interfacial tensions and improve the contact of spray solutions or suspensions with treated surfaces.

Preferably the wetting agent is present in an amount of 0 to 20% by weight, more preferably 1 to 5% by weight, based on the dry weight of the coating.

The composition of the invention may be additionally enteric coated. By enteric coated or coating is meant a pharmaceutically acceptable coating preventing the release of the active agent in the stomach and allowing the release in the upper part of the intestinal tract. The enteric coating may be added as an overcoat upon the modified release coating.

The preferred enteric coating for the composition of the invention comprises a film-forming agent selected from e.g. cellulose acetate phthalate; cellulose acetate trimellitate; methacrylic acid copolymers, e.g. copolymers derived from methylacrylic acid and esters thereof, containing at least 40% methylacrylic acid; hydroxypropyl methylcellulose phthalate; hydroxypropylmethylcellulose acetate succinate or Polyvinylacetatephthalate,

Typical cellulose acetate phthalates have an acetyl content of 17-26% and a phthalate content of from 30-40% with a viscosity of ca. 45-90 cP. An example of an appropriate cellulose acetate phthalate is the marketed product CAP (Eastman Kodak, Rochester N.Y., USA or Aquacoat CPD from FMC Biopolymer).

Typical cellulose acetate trimellitates have an acetyl content of 17-26%, a trimellityl content from 25-35% with a viscosity of ca. 15-20 cS. An example of an appropriate cellulose acetate trimellitate is the marketed product CAT (Eastman Kodak Company, USA).

Methacryclic acid copolymers include preferably copolymers derived from methylacrylic acid and esters thereof, containing at least 40% methylacrylic acid, more preferably those of molecular weight above 100,000 Daltons based on, e.g. methylacrylate and methyl or ethyl methylacrylate in a ratio of about 1:1. Typical products include Eudragit L, e.g. L 100-55, L30 D marketed by Rohm GmbH, Darmstadt, Germany or Acryl-Eze from Colorcon, Kollicoat MAE 30 DP from BASF.

HPMC-phthalates and HPMC-acetate succinate are as defined hereinabove. Examples of suitable HPMC- phthalates are HP50 or HP55. Examples of suitable hydroxypropylmethylcellulose acetate succinate may be used as known under the trademark Aqoat LF or Aqoat MF (both Shin-Etsu).

The enteric coating may further comprise further components such as a plasticizer, e.g. triacetine, triethylcitrate, diethylsebacate, polyethyleneglycol 3000, 4000 or 6000, acetyltriethylcitrate, acetyltributylcitrate, or diethylphthalate, and/or antisticking agents, e.g. colloidal silicon dioxide, an synthetic amorphous silicic acid such as Syloid 244 FP, talc, or glycerine monostearate. The coating may further comprise, especially in aqueous dispersions, one or more thickening agents to avoid sedimentation of suspended excipients, e.g. HPMC 3cps or HPMC 6 cps.

Preferably the enteric-coating may further comprise a film-forming agent, e.g. cellulose acetate phthalate, cellulose acetate trimellitate, methacrylic acid copolymer, hydroxypropyl methylcellulose phthalate or hydroxypropylmethylcellulose acetate succinate, polyvinylacetatephthalate. The amount of the film-forming agent may be from 50 to 95% by weight, based on the total weight of the enteric coating, more preferably 60 to 80% by weight. The plasticizer and/or the antisticking agent, if present in the enteric-coating, may be e.g. as disclosed above for the modified release coat, e.g. in the amount as indicated above for the modified release coat.

According to the invention, the drug substance is preferably present in the composition of the invention in an amount of 1 to 99% by weight, based on the total weight of the core (i.e. excluding the coating). In particular when the composition of the invention is in the form of, small tablets, minitablets, pellets, beads or granules, the drug substance is preferably present in an amount of 1 to 95% by weight, more preferably 20 to 90%, most preferably 30 to 80% by weight, based on the total weight of the core (i.e. excluding the coating). When the composition of the invention is in the form of particles, or microparticles the drug substance is preferably present in an amount of 1 to 95% by weight, more preferably to 50-95%, most preferably to 70-90 % by weight, based on the total weight of the core (i.e. excluding the coating).

The composition of the invention may contain one or more excipients or diluents, e.g. as hereinafter disclosed.

A preferred group of multiparticulates of component a) and /or component b) according to the invention are those having an effective average particle size of less than about 1000 µm, preferably between about 10 and 800 µm, more preferably between 30 and 500 µm. The drug microparticles are preferably combined with one or more pharmaceutically acceptable excipients to form a drug excipient matrix, e.g. with ethylcellulose or a methacrylic acid copolymer and a stabilizer, e.g. colloidal silica, to form the microparticle drug core, for instance by spray-drying, fluid-bed drying or precipitation techniques. Crystalline particles, e.g. in a size range between 1 and 200 micron (µm), may also be prepared by means of high pressure homogenization of a suspension of unmilled crystalline drug crystals in any fluid in which the drug substance is sparsely soluble, such as organic solvents, e.g. cyclohexane.

These microparticulate drug suspensions may be directly coated with the aid of a polymer, or embedded in a polymer matrix, e.g. by adding the polymer and dissolving it in the homogenized suspension which is subsequently spray dried or spray layered. Preferably polymers used are Ethylcellulose or acrylic and methacrylic copolymers containing quaternary ammonium groups.

The precipitation techniques may also include the coacervation techniques, e.g. to separate a liquid phase of a coating material from a polymeric solution and wrapping of that phase as a uniform layer around suspended core particles. The resulting microparticles may be collected by filtration or centrifugation, washed with an appropriate solvent, and subsequently dried by standard techniques such as spray drying or fluidized bed drying.

These drug particles may then be further coated with modified release coating ingredients as disclosed herein, and optionally a stabilizer, e.g. colloidal silica. The modified release coating may be prepared for instance by fluid-bed coating and/or granulation or precipitation techniques. The proper coating technology can be selected by a person skilled in the art.

The resulting coated drug particles may optionally be combined with a diluent, e.g. as disclosed hereinafter, for example lactose, mannitol or sucrose, a lubricant, e.g. as disclosed hereinafter, for instance magnesium stearate and dispensed in a capsule or a sachet or compressed into tablets.

In another embodiment component a) and/or component b) may optionally be combined with a binder or optionally with diluent and a binder, e.g. as disclosed herein after, and formed into granules, e.g. using a technique such as high or low shear granulation or fluid bed granulation to form the granule drug core. The granules obtained may then be coated with modified release coating ingredients, e.g. as disclosed herein, and e.g. dispensed in a capsule or a sachet. The granule drug core typically has a mean width of diameter of from 0.05 to 2mm or preferably from 0.1 to 2mm, or more preferably of from 0.15 to 1.5mm . The amount of drug substance present in the core may be from 1 to 95% or preferably form 20 to 90%, or more preferably from 50 to 90% by weight, based on the total weight of the granule drug core (i.e. excluding the coating).

Drug particles were the drug is in the form of crystals, amorphous particles or a mixture thereof can also be used for subsequent coating.

In another embodiment component a) and/or component b) may optionally be combined with one or more pharmaceutically acceptable extrusion aid(s), e.g. microcrystalline cellulose, an amylose pregelled starch, etc., binder(s), e.g. as herein disclosed, or diluents, e.g. as herein disclosed, and formed into pellets, e.g. using a technique such as extrusion spheronisation, direct pelletisation/high or low shear granulation, fluid bed granulation or spray drying/melt concealing to form the pellet drug core. The pellets obtained may be coated with modified release coating ingredients, e.g. as herein disclosed and dispensed in a capsule or a sachet. The pellet drug core typically has a width of diameter of from 0.2 to 2mm, preferably of from 0.5 to 1.4mm. The amount of drug substance present in the core may be from 1 to 95% or preferably form 20 to 90%, or more preferably from 50 to 90% by weight, based on the total weight of the granule drug core (i.e. excluding the coating).

In another embodiment, the drug optionally in combination with a pharmaceutically acceptable binder, may be layered onto the surface of a pharmaceutically acceptable seed, typically a particle (e.g. a sphere) of sucrose, lactose, mannitol, starch, microcrystalline cellulose or any combination thereof, to form the bead drug core. Such layering may be solution layering or powder layering. Such a pharmaceutically acceptable seed is preferably a non-pareil sugar/starch sphere of 18-20 mesh, 25-30 mesh or 35-40 mesh, most preferably a non-pareil sugar starch sphere of 25-30 mesh or Cellets, i.e. microcrystalline cellulose beads e.g. from Pharmatrans Sanaq AG, in the size range of 100-1000 µm, more preferably 100-200 and 200-355 µm. The beads obtained may be coated with modified release coating ingredients, e.g. as herein disclosed, and dispensed in a capsule or a sachet or further processed by layering of another drug. The bead drug core typically has a width of diameter of from 0.2 to 2 mm, preferably of from 0.5 to 1.4mm. The amount of drug substance present in the core may be from 1 to 95% or preferably form 20 to 90%, or more preferably from 50 to 90% by weight, based on the total weight of the granule drug core (i.e. excluding the coating).

In a further embodiment, coated drug particle or coated granules or coated pellet drug cores may optionally be combined with pharmaceutically acceptable ingredients, e.g. a diluent, binder, lubricant, e.g. as herein disclosed, well known to the skilled person to form tablets and or small tablets which disintegrate in the stomach and release the coated drug particles, or coated pellets or coated granules.

Component a) and /or component b) may be granulated prior to the preparation of minitablets or small tablets.

The tablets comprise the drug , i.e. component a) and / or component b), a binder and a filler. This granulate may be compressed into tablets /minitablets optionally with additional filler, binder, disintegrant and lubricant.

Examples of filler, binder, disintegrant and lubricant are as described below.

Suitable fillers include, without limitation, microcrystalline cellulose (e.g., Avicel PH 101, PH 102, PH105), mannitol, sucrose or other sugars or sugar derivatives, Calcium hydrogen phosphate, low-substituted hydroxypropyl cellulose (L-HPC), hydroxypropyl cellulose, hydroxyethyl cellulose, hydroxypropyl methyl cellulose, and combinations thereof, preferably, microcrystalline cellulose, e.g. Avicel PH102, or similar products available under the registered trade marks AVICEL, FILTRAK, HEWETEN or PHARMACEL.

Suitable binders include, without limitation, polyvinylpyrrolidone (PVP), such as e.g., PVP K 30 or PVP90, polyethylene glycols (PEG), e.g., PEG 4000, hydroxypropylmethyl cellulose, hydroxypropyl cellulose, both preferably of low viscosity, e.g. viscosity grades 3 or 6 cps, pregelatinized starch and combinations thereof..

Suitable lubricants include, without limitation, magnesium stearate, aluminum or calcium silicate, stearic acid, cutina, PEG 4000-8000, talc and combinations thereof, preferably magnesium stearate.

Suitable disintegrants include, without limitation, carboxymethylcellulose calcium (CMC-Ca), carboxymethylcellulose sodium (CMC-Na), crosslinked PVP (e.g. CROSPOVIDONE, POLYPLASDONE or KOLLIDON XL), alginic acid, sodium alginate and guar gum, most preferably crosslinked PVP (CROSPOVIDONE), crosslinked CMC (Ac-Di-Sol), carboxymethylstarch-Na (PIRIMOJEL and EXPLOTAB). A most preferred disintegrant is crosslinked PVP (PVPPXL.

Suitable glidants include, without limitation, colloidal silicon dioxide (e.g., Aerosil 200), magnesium trisilicate, powdered cellulose, starch, talc and combinations thereof.

The composition of the invention preferably comprises the filler in an amount of 0 to 50% by weight, based on the total weight of the uncoated tablet composition, more preferably 3 to 30% by weight, most preferably 5 to 15% by weight.

Preferably the composition of the invention comprises the binder in an amount of 0 to 30% by weight, based on the total weight of the uncoated composition, more preferably 1 to 20% by weight, most preferably 3 to 15% by weight.

The composition of the invention preferably comprises the disintegrant in an amount of up to 20% by weight, based on the total weight of the uncoated composition, more preferably 5 to 15%.

The composition of the invention preferably comprises the glidant in an amount of up to 5% by weight, based on the total weight of the uncoated composition, more preferably 0.1 to 2%, most preferably 0.2 to 0.5%

The composition of the invention preferably comprises the lubricant in an amount of up to 5% by weight, based on the total weight of the uncoated composition, more preferably 0.5 to 2%.

Procedures which may be used to prepare and/or to coating the compositions of the invention may be conventional or known in the art or based on such procedures e.g. those described in L. Lachman et al. The Theory and Practice of Industrial Pharmacy, 3rd Ed, 1986, H. Sucker et al, Pharmazeutische Technologie, Thieme, 1991, Hager's Handbuch der pharmazeutischen Praxis, 4th Ed. (Springer Verlag, 1971) and Remington's Pharmaceutical Sciences, 13th Ed., (Mack Publ., Co., 1970) or later editions. Minitablets may e.g. manufactured on a standard rotary tabletting machine.

The resulting formulations in accordance with the present invention show the following advantages:
- formulations approaching or reaching bioequivalence are achieved in contrast to monolithic tablets;
- The formulation of pharmaceutical oral fixed dose combinations with sufficient hardness, resistance to friability, disintegration time etc. is possible;
- The sticking tendency and poor flow of the drug substance is reduced to a minimum;
- A robust manufacturing process is achieved;
- Scale-up of formulation and process resulting in a reproducible performance is achieved; and
- Sufficient stability to achieve a reasonable shelf life is achieved.

The invention likewise relates to a process for the preparation of pharmaceutical oral fixed dose combinations as described herein above. Such pharmaceutical oral fixed dose combination may be produced by working up components as defined herein above in the appropriate amounts, to form unit pharmaceutical oral fixed dose combinations.

The pharmaceutical oral fixed dose combinations of the present invention are useful for lowering the blood pressure, either systolic or diastolic or both. The conditions for which the instant invention is useful include, without limitation, hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction (such as Alzheimer's) and stroke, headache and chronic heart failure.

The present invention likewise relates to a method of treating hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure comprising administering to an animal, including human patient, in need of such treatment a therapeutically effective pharmaceutical oral fixed dose combination according to the present invention.

The present invention likewise relates to the use of a pharmaceutical oral fixed dose combination according to the present invention for the manufacture of a medicament for the treatment of hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure.

The present invention likewise relates to a pharmaceutical composition for the treatment of hypertension (whether of the malignant, essential, reno-vascular, diabetic, isolated systolic, or other secondary type), congestive heart failure, angina (whether stable or unstable), myocardial infarction, artherosclerosis, diabetic nephropathy, diabetic cardiac myopathy, renal insufficiency, peripheral vascular disease, left ventricular hypertrophy, cognitive dysfunction, e.g., Alzheimer's, stroke, headache and chronic heart failure, comprising a pharmaceutical oral fixed dose combination according to the present invention.

Ultimately, the exact dose of the active agent and the particular formulation to be administered depend on a number of factors, e.g., the condition to be treated, the desired duration of the treatment and the rate of release of the active agent. For example, the amount of the active agent required and the release rate thereof may be determined on the basis of known in vitro or in vivo techniques, determining how long a particular active agent concentration in the blood plasma remains at an acceptable level for a therapeutic effect.

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore, the Examples herein are to be construed as merely illustrative and not a limitation of the scope of the present invention in any way.

### Examples :

### Example 1: Drug Product

**Table 1 Composition of fixed-dose combination of Aliskiren and Valsartan as 300/320 mg film-coated tablet**

| **Ingredient** | **Quantity mg** | **Function** | **Reference to standards** |
|---|---|---|---|
| **Aliskiren pellets** | | | |
| Aliskiren hemifumarate | 331.5* | Active pharmaceutical ingredient | Novartis |
| Cellulose, microcrystalline / | 19.6 | Carrier | Ph. Eur. / NF microcrystalline cellulose |
| **Ethylcellulose** | 42.4 | Film-forming agent | Ph. Eur. / NF |
| Anhydrous ethanol / dehydrated alcohol** | --- | solvent | Ph. Eur. / USP |
| Hydroxypropylcellulose | 8.3 | Pore former | Ph. Eur. /NF |
| **Aliskiren pellets weight** | **402** | | |
| **Valsartan pellets** | | | |
| Valsartan | 320.0 | Active pharmaceutical ingredient | Novartis |
| Cellulose, microcrystalline / microcrystalline cellulose | 32.0 | Binder | Ph. Eur. /NF |
| Crospovidone | 48.0 | Disintegrant | Ph. Eur. /NF |
| Water, purified / Purified water** | --- | Granulation fluid | Ph. Eur. / USP |
| **Valsartan pellets weight** | **400** | | |
| **Tablet core** | | | |
| Aliskiren pellets*** | 402 | | |
| Valsartan pellets*** | 400 | | |
| Cellulose, microcrystalline / | 85 | Diluent | Ph. Eur. / NF |
| microcrystalline cellulose**** | | | |
| Crospovidone | 100.0 | Disintegrant | Ph. Eur. /NF |
| Silica, colloidal anhydrous / colloidal silicon dioxide NF | 3.0 | Glidant | Ph. Eur. / |
| Magnesium stearate | 10.0 | Lubricant | Ph. Eur. /NF |
| **Core tablet weight** | **1000.0** | | |
| **Coating** | | | |
| Basic coating premix - White ***** | 36.80 | Film-coating polymer | Novartis monograph |
| Basic coating premix - Red ***** | 0.09 | Film-coating polymer | Novartis monograph |
| Basic coating premix - Black ***** | 0.11 | Film-coating polymer | Novartis monograph |
| Water, purified / Purified water** | --- | Solvent | Ph. Eur. / USP |
| **Total film-coated tablet weight** | **1037.0** | | |

| | | | |
|---|---|---|---|
| * Corresponds to 300 mg base ** Removed during processing *** The actual amount added is based on assay for content value of the active pharmaceutical ingredient of the pellets to ensure that the correct amount (label claim) of drug is present in th tablet. The presented amounts assume that assay for content is 100% **** Cellulose, microcrystalline compensates the variable amount of pellets to keep the total amount of pellets and cellulose, microcrystalline on 887.00 mg ***** Coating premix is a commercially available material (Opadry, Colorcon Ltd., UK) containing one or more iron oxides and/ortitanium dioxide as the coloring agent(s). The individual components meet pharmacopoeial and/or international standards as applicable. | | | |

The manufacturing process can be split in two parts. The first part is the production of 2 pharmaceutical intermediates, pellets coated with Aliskiren and creating pellets from Valsartan. The second step is the mixing of the 2 intermediated with other Tabletting excipients, compressing tablet cores and subsequent film-coating of these cores.

**Table 2 Manufacturing formula for a subbatch of approx. 1300 g of Aliskiren single layered pellets**

| **Ingredient** | **Amount per batch (g)** |
|---|---|
| **Core** | |
| Cellulose, microcrystalline | 300.0 |
| **Coating**** | |
| Aliskiren hemifumarate | 997.5* |
| Ethylcellulose | 52.5 |
| Anhydrous ethanol | 4200.0*** |

| | |
|---|---|
| *Salt factor of 1.105. Theoretical weight of the drug substance based on the dried substance (100%) and 100% assay value **Including (5)% overage to compensate for losses during the coating process ***Removed during processing | |

**Table 3 Manufacturing formula for a subbatch of approx. 1300 g double layered Aliskiren pellets**

| **Ingredient** | **Amount per batch (g)** |
|---|---|
| **Core** | |
| Aliskiren single layered pellets | 300.0 |
| **Coating*** | |
| Aliskiren hemifumarate | 997.5 |
| Ethylcellulose | 52.5 |
| Anhydrous ethanol | 4200.0** |

| | |
|---|---|
| *Including (5)% overage to compensate for losses during the coating process **Removed during processing | |

**Table 4 Manufacturing formula for a subbatch of approx. 1144.5 g of Aliskiren pellets**

| **Ingredient** | **Amount per batch (g)** |
|---|---|
| **Core** | |
| Aliskiren double layered pellets | 1050.0 |
| **Coating*** | |
| Ethylcellulose | 74.4 |
| Hydroxypropyl cellulose | 24.8 |
| Anhydrous ethanol | 1885.3** |

| | |
|---|---|
| *Including (5)% overage to compensate for losses during the coating process **Removed during processing | |

**Table 5 Manufacturing formula for a subbatch of approx. 2500 g of Valsartan pellets**

| **Ingredient** | **Amount per batch (g)** |
|---|---|
| **Granulate** | |
| Valsartan | 2000.0 |
| Crospovidone | 300.0 |
| Cellulose, microcrystalline | 200.0 |
| Water, purified | 2000.0*** |
| **Total, granulate** | 2500.0 |
| **Spheronisation** | |
| Water, purified | 2000.0*** |
| **Total batch weight** | 2500.0 |

| | |
|---|---|
| *Prepared in excess, actual amount is documented **Removed during processing | |

### Manufacturing process

### Aliskiren Pellets

1. Load a suitable fluid bed coater with cellulose, microcrystalline cellets.
2. Prepare coating fluid by loading a vessel with anhydrous ethanol. While stirring until dissolved add subsequently ethylcellulose and then Aliskiren hemifumarate.
3. Spray coat the pre-heated cellets with coat solution prepared in step 2, followed by a drying step. These coated cellets are sieved and dried again (Aliskiren single layered pellets).
4. Prepare coating fluid by loading a vessel with anhydrous ethanol. While stirring until dissolved add subsequently ethylcellulose and then Aliskiren hemifumarate.
5. Load a suitable fluid bed coater with Aliskiren single layered pellets (step 3).
6. Spray coat the pre-heated Aliskiren single layered pellets with coat solution prepared in step 4, followed by a drying step. These coated pellets are sieved and dried again (Aliskiren double layered pellets).

Steps 4 to 6 may be repeated 3 to 4 times.
7. Prepare coating fluid by loading a vessel with anhydrous ethanol. While stirring until dissolved add subsequently ethylcellulose and then hydroxypropylcellulose.
8. Load a suitable fluid bed coater with Aliskiren double layered pellets (step 6).
9. Spray coat the pre-heated Aliskiren double layered pellets with coat solution prepared in step 7, followed by a drying step. These coated pellets are sieved and dried again.

Steps 7 to 9 may be repeated to coat each batch of the previous coating step.
10. Mix the subbatches using a suitable blender.

### Valsartan Pellets

1. Load a suitable granulator with cellulose, microcrystalline, crospovidone and Valsartan and mix.
2. Granulate the mix from step 1 while spraying water.
3. Transfer granulate from step 2 into a suitable fluid bed coater and granulate until the suitable particle size is reached.
4. Pellets from step 3 are dried and subsequently sieved.

Steps 1 to 4 may be repeated.
5. Mix subbatches using a suitable blender.

### Compression and coating

1. Load sufficient amount (based on assay of active ingredient content) of Aliskiren pellets and Valsartan pellets in a suitable blender.
2. Add pre-sieved cellulose microcrystalline, crospovidone and silicone, colloidal anhydrous.
3. Mix ingredients from step 1 and 2.
4. Add pre-sieved magnesium stearate and continue mixing.
5. Compress the final blend using a suitable tablet press to obtain the tablet cores.
6. Prepare the aqueous coating suspension by adding the commercially available coating premixes to stirred water, and film coat the tablets using a suitable film-coater.

Aliskiren pellets and Valsartan pellets are pharmaceutical intermediates and are controlled by monitoring equipment settings and in process controls. The result of the analytical determination of the drug substance content in the respective intermediates are used to calculate the right amount of intermediate to be added to the tablet matrix to ensure that the correct content of active ingredient in the final film-coated tablet.

The following results describe the quality of the product:

**Table 6 Analytical results of Example 1**

| **Intermediates** | | |
|---|---|---|
| Aliskiren content in pellets | 408.5 | mg |
| Valsartan content in pellets | 399.6 | mg |
| Tablet core hardness | 139 -156 | N |
| Tablet core average weight | 993 -1006 | mg |
| Tablet core friability | > 0.4 | % |
| Tablet core disintegration time | < 20 | Min. |
| Tablet core width * breadth | 18.8 * 9.5 | mm |
| Tablet core thickness | Approx. 7.6 | Mm |
| Tablet core shape | Oblong | -- |

| **Film-coated tablet** | | |
|---|---|---|
| Aliskiren content | 102.4 | % |
| Valsartan content | 98.0 | % |
| Aliskiren and Valsartan sum of degradation products | 0.2 | % |
| Content uniformity of Aliskiren | 99.6 +/- 4.6 | % |
| Content uniformity of Valsartan | 97.4 +/- 1.8 | % |
| Dissolution Aliskiren pH 4.5 after 10min | 35 | % |
| Dissolution Aliskiren pH 4.5 after 20min | 60 | % |
| Dissolution Valsartan pH 4.5 after 30min | 55 | % |
| Dissolution Valsartan pH 4.5 after 60min | 69 | % |

| | | |
|---|---|---|
| Dissolution pH 4.5 is performed as described in example 2. | | |

Stability testing of this formulation revealed that this product is stable at 30°C/65%rh for at least 6 month. If tested for longer period than 6 month very probably a longer shelf live than 6 month could be expected.

### Example 2: DISSOLUTION TESTING

The dissolution property of the formulations in accordance with the present invention were confirmed as follows.

### For paddle method at pH 4.5:

The assembly consists of the following: a covered vessel made of glass or other inert, transparent material; a motor, and a paddle formed from a blade and shaft as the stirring element. The vessel is partially immersed in a suitable water bath of any convenient size or placed in a heating jacket. The water bath or heating jacket permits holding the temperature inside the vessels at 37 ± 0.5° during the test and keeping the bath fluid in constant, smooth motion. No part of the assembly, including the environment in which the assembly is placed, contributes significant motion, agitation, or vibration beyond that due to the smoothly rotating stirring element. Apparatus that permits observation of the specimen and stirring element during the test is has the following dimensions and capacities: the height is 160 mm to 210 mm and its inside diameter is 98 mm to 106 mm. Its sides are flanged at the top. A fitted cover may be used to retard evaporation.

The shaft is positioned so that its axis is not more than 2 mm at any point from the vertical axis of the vessel and rotates smoothly without significant wobble. The vertical center line of the blade passes through the axis of the shaft so that the bottom of the blade is flush with the bottom of the shaft. The design of the paddle is as shown in USP <711 >, Fig. 2. The distance of 25 ± 2 mm between the blade and the inside bottom of the vessel is maintained during the test. The metallic or suitably inert, rigid blade and shaft comprise a single entity. A suitable two-part detachable design may be used provided the assembly remains firmly engaged during the test. The paddle blade and shaft may be coated with a suitable inert coating. The dosage unit is allowed to sink to the bottom of the vessel before rotation of the blade is started. A small, loose piece of nonreactive material such as not more than a few turns of wire helix may be attached to dosage units that would otherwise float. Other validated sinker devices may be used.

1 L of the Dissolution Medium* is placed in the vessel of the apparatus, the apparatus is assembled, the Dissolution Medium is equilibrated to 37 ± 0.5°, and the thermometer is removed. 1 dosage form (e.g. tablet or capsule) is placed on the apparatus, taking care to exclude air bubbles from the surface of the dosage-form unit, and immediately the apparatus is operated at a rate of 75±3 rpm or 100±3rpm depending on the pH. Within the time interval specified (e.g. 10, 20, 30, 45, 60, 90 and 120 min.), or at each of the times stated, a specimen (≥ 1 ml) is withdrawn from a zone midway between the surface of the Dissolution Medium and the top of the rotating blade, not less than 1 cm from the vessel wall. [NOTE-the aliquots withdrawn for analysis are replaced with equal volumes of fresh Dissolution Mediums at 37° or, where it can be shown that replacement of the medium is not necessary, the volume change is corrected in the calculation. The vessel is kept covered for the duration of the test, and the temperature of the mixture under test at suitable times is verified.] . The specimen is filtered through a suitable filter, e.g. a 0.45 µm PVDF filter (Millipore) and the first mls (2 to 3 ml) of the filtrate are discarded. The analysis is performed by HPLC or UV detection. The test is repeated at least 6 times, with additional dosage form units.

* Dissolution medium for pH 4.5: 1 L of a buffered aqueous solution, adjusted to pH 4.5 ± 0.05 (0.1 M Phosphate buffer solution obtained by dissolving 13.61 g of potassium hydrogen phosphate in 750 ml of deionized water and diluted to 1 L with deionized water)

The examples of multiparticulate systems prepared according to the present invention all had the required dissolution characteristics as set forth in the claims of the present invention. The results are shown in Example 1.

### Example 3: BIOEQUIVALENCE TESTING

The bioavailability of the pharmaceutical oral fixed dose combinations of the present invention was compared with that of the corresponding free dose combinations. The test (fixed dose combination) and the reference (free dose combination) dosage forms were administered orally to the subjects, and plasma samples were collected over a 48-hour time period. The plasma samples were analyzed for concentration of Valsartan and Aliskiren. Statistical comparison was performed on the maximum plasma concentration (Cmax) achieved with the test and reference and on the area under the plasma concentration vs. time curve (AUC).

The examples of pharmaceutical oral fixed dose combinations of the present invention of Valsartan and Aliskiren (320/300 mg) made in accordance with the present invention was compared with a free dose combination of 320 mg Valsartan (2 times 160mg capsules) and 300mg Aliskiren tablets in an open-label, randomized, single dose, three period, crossover study in healthy human volunteers. The bioavailability of the fixed dose combination tablets of Valsartan and Aliskiren were compared with the free dose combination. The results are shown in the table below.

### Reference:

- Aliskiren tablet 300mg
- Valsartan: hard gelatine capsule 160mg (2 capsules are given as single dose to the subjects)

Both reference products are marketed products by Novartis Pharma AG, Switzerland
Test: Formulation described in Example 1
Number of subjects: 42 enrolled

**Table 7 Results of human pharmacokinetic study for Aliskiren**

| **Parameter** | **Treatment** | **Geometric** | **Geometric Mean Ratio** | **90% Cl** |
|---|---|---|---|---|
| AUC0-inf (ng.hr/mL) | | | | |
| | Example1 | 1090 | 0.82 | (0.69,0.98) |
| | | | | |
| | Reference | 1325 | -- | -- |
| AUC0-tlast (ng.hr/mL) | | | | |
| | Example1 | 997 | 0.81 | (0.69, 0.96) |
| | | | | |
| | Reference | 1225 | -- | -- |
| Cmax (ng/mL) | | | | |
| | Example1 | 156 | 0.60 | (0.46, 0.78) |
| | | | | |
| | Reference | 260 | -- | -- |

**Table 7 Results of human pharmacokinetic study for Valsartan**

| **Parameter** | **Treatment** | **Geometric Mean** | **Geometric Mean Ratio** | **90% Cl** |
|---|---|---|---|---|
| AUC0-inf (ng.hr/mL) | | | | |
| | Example1 | 37636 | 1.07 | (0.96, 1.18) |
| | | | | |
| | Reference | 35259 | -- | -- |
| AUC0-tlast (ng.hr/mL) | | | | |
| | Example1 | 37288 | 1.07 | (0.97,1.19) |
| | | | | |
| | Reference | 34823 | | |
| Cmax (ng/mL) | | | | |
| | Example1 | 4999 | 1.09 | (0.95, 1.26) |
| | | | | |
| | Reference | 4580 | -- | -- |

## Claims

1. A pharmaceutical oral fixed dose combination comprising
a) a therapeutically effective amount of Aliskiren, or a pharmaceutically acceptable salt thereof,
b) a therapeutically effective amount of Valsartan, or a pharmaceutically acceptable salt thereof,
wherein the pharmaceutical oral fixed dose combination shows an in vitro dissolution of component (a) of 60% or less after 10 minutes and 95% or less after 20 minutes, and a dissolution profile of component (b) of 25 % or more after 30 minutes, and 45% or more after 60 minutes at pH 4.5.

2. The pharmaceutical oral fixed dose combination according to claim 1, having an asynchronous release profile of component (a) and component (b).

3. The pharmaceutical oral fixed dose combination according to claim 1 or 2, having a continuous release of both components (a) and (b).

4. The pharmaceutical oral fixed dose combination according to any of the preceding claims, wherein the release of component (a) is modified by delaying the time of release or by slowing down the release rate.

5. The pharmaceutical oral fixed dose combination according to any of the preceding claims, wherein component (b) exhibits immediate release.

6. The pharmaceutical oral fixed dose combination according to any of the preceding claims, wherein the pharmaceutical oral fixed dose combination is a solid dosage form.

7. The pharmaceutical oral fixed dose combination according to any of the preceding claims, wherein component (a) is physically separated from component (b).

8. The pharmaceutical oral fixed dose combination according to any of the preceding claims, in the form of multiparticulates, comprising particulates of different populations of component a) and/or component b) respectively.

9. The pharmaceutical oral fixed dose combination according to claim 8, wherein the component a) containing particulates contain a modified release coating.

10. The pharmaceutical oral fixed dose combination according to claim 8 or 9 in the form of a tablet or film-coated tablet.

11. The pharmaceutical oral fixed dose combination according to any of the preceding claims, wherein component (a) is present in an amount ranging from 75 to 300 mg of the free base per unit dosage form.

12. The pharmaceutical oral fixed dose combination according to any of the preceding claims, wherein component (b) is present in an amount ranging from 80 to 320 mg per unit dosage form.

13. A pharmaceutical oral fixed dose combination according to any of the preceding claims for use in the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure, in particular hypertension.

## Patentansprüche

1. Orale pharmazeutische fixierte Dosiskombination, die
a) eine therapeutisch wirksame Menge von Aliskiren oder einem pharmazeutisch akzeptablen Salz hiervon,
b) eine therapeutisch wirksame Menge von Valsartan oder einem pharmazeutisch akzeptablen Salz hiervon umfasst,
wobei die orale pharmazeutische fixierte Dosiskombination eine *in* vitro-Auflösung der Komponente (a) von 60 % oder weniger nach 10 Minuten und 95 % oder weniger nach 20 Minuten und ein Auflösungsprofil der Komponente (b) von 25 % oder mehr nach 30 Minuten und 45 % oder mehr nach 60 Minuten bei einem pH-Wert von 4,5 zeigt.

2. Orale pharmazeutische fixierte Dosiskombination nach Anspruch 1 mit einem asynchronen Freisetzungsprofil der Komponente (a) und der Komponente (b).

3. Orale pharmazeutische fixierte Dosiskombination nach Anspruch 1 oder 2 mit einer kontinuierlichen Freisetzung sowohl der Komponente (a) als auch der Komponente (b).

4. Orale pharmazeutische fixierte Dosiskombination nach einem der vorhergehenden Ansprüche, wobei die Freisetzung der Komponente (a) durch Verzögern der Zeit der Freisetzung oder durch Verlangsamen der Freisetzungsrate modifiziert ist.

5. Orale pharmazeutische fixierte Dosiskombination nach einem der vorhergehenden Ansprüche, wobei die Komponente (b) eine unmittelbare Freisetzung zeigt.

6. Orale pharmazeutische fixierte Dosiskombination nach einem der vorhergehenden Ansprüche, wobei die orale pharmazeutische fixierte Dosiskombination eine feste Dosierungsform ist.

7. Orale pharmazeutische fixierte Dosiskombination nach einem der vorhergehenden Ansprüche, wobei die Komponente (a) von der Komponente (b) physikalisch getrennt ist.

8. Orale pharmazeutische fixierte Dosiskombination nach einem der vorhergehenden Ansprüche in Form von Mehrfachpartikeln, die Partikel unterschiedlicher Populationen der Komponente (a) und/oder der Komponente (b) umfassen.

9. Orale pharmazeutische fixierte Dosiskombination nach Anspruch 8, wobei die Partikel enthaltende Komponente (a) einen für eine modifizierte Freisetzung sorgenden Überzug enthält.

10. Orale pharmazeutische fixierte Dosiskombination nach Anspruch 8 oder 9 in Form einer Tablette oder einer Filmtablette.

11. Orale pharmazeutische fixierte Dosiskombination nach einem der vorhergehenden Ansprüche, wobei die Komponente (a) in einer Menge im Bereich von 75 bis 300 mg der freie Base pro Dosierungsformeinheit vorhanden ist.

12. Orale pharmazeutische fixierte Dosiskombination nach einem der vorhergehenden Ansprüche, wobei die Komponente (b) in einer Menge in einem Bereich von 80 bis 320 mg pro Dosierungsformeinheit vorhanden ist.

13. Orale pharmazeutische fixierte Dosiskombination nach einem der vorhergehenden Ansprüche zur Verwendung bei der Behandlung von Bluthochdruck, dekompensierter Herzinsuffizienz, Angina, Myokardinfarkt, Atherosklerose, diabetischer Nephropathie, diabetischer Herzmyopathie, Niereninsuffizienz, peripherer Gefäßerkrankung, Linksherzhypertrophie, kognitiver Fehlfunktion, Herzschlag, Kopfschmerz und chronischer Herzinsuffizienz, insbesondere Bluthochdruck.

## Revendications

1. Combinaison de doses fixes pharmaceutiques orales comprenant
a) une quantité thérapeutiquement efficace d'Aliskiren, ou de l'un de ses sels pharmaceutiquement acceptables,
b) une quantité thérapeutiquement efficace de Valsartan, ou de l'un de ses sels pharmaceutiquement acceptables,
où la combinaison de doses fixes pharmaceutiques orales présente une dissolution in vitro du composant (a) de 60% ou moins après 10 minutes et de 95% ou moins après 20 minutes, et un profil de dissolution du composant (b) de 25% ou plus après 30 minutes, et de 45% ou plus après 60 minutes à pH 4,5.

2. Combinaison de doses fixes pharmaceutiques orales selon la revendication 1, ayant un profil de libération asynchrone du composant (a) et du composant (b).

3. Combinaison de doses fixes pharmaceutiques orales selon la revendication 1 ou 2, ayant une libération continue des deux composants (a) et (b).

4. Combinaison de doses fixes pharmaceutiques orales selon l'une quelconque des revendications précédentes, dans laquelle la libération du composant (a) est modifiée en retardant le moment de libération ou en ralentissant la vitesse de libération.

5. Combinaison de doses fixes pharmaceutiques orales selon l'une quelconque des revendications précédentes, dans laquelle le composant (b) présente une libération immédiate.

6. Combinaison de doses fixes pharmaceutiques orales selon l'une quelconque des revendications précédentes, où la combinaison de doses fixes pharmaceutiques orales est une forme posologique solide.

7. Combinaison de doses fixes pharmaceutiques orales selon l'une quelconque des revendications précédentes, dans laquelle le composant (a) est séparé physiquement du composant (b).

8. Combinaison de doses fixes pharmaceutiques orales selon l'une quelconque des revendications précédentes, sous forme de multiparticules, comprenant des particules de différentes populations du composant a) et/ou du composant b) respectivement.

9. Combinaison de doses fixes pharmaceutiques orales selon la revendication 8, dans laquelle les particules contenant le composant a) contiennent un enrobage de libération modifiée.

10. Combinaison de doses fixes pharmaceutiques orales selon la revendication 8 ou 9 sous forme d'un comprimé ou d'un comprimé pelliculé.

11. Combinaison de doses fixes pharmaceutiques orales selon l'une quelconque des revendications précédentes, dans laquelle le composant (a) est présent en une quantité allant de 75 à 300 mg de la base libre par forme posologique unitaire.

12. Combinaison de doses fixes pharmaceutiques orales selon l'une quelconque des revendications précédentes, dans laquelle le composant (b) est présent en une quantité allant de 80 à 320 mg par forme posologique unitaire.

13. Combinaison de doses fixes pharmaceutiques orales selon l'une quelconque des revendications précédentes à utiliser dans le traitement de l'hypertension, de l'insuffisance cardiaque congestive, de l'angine, de l'infarctus du myocarde, de l'athérosclérose, de la néphropathie diabétique, de la myopathie cardiaque diabétique, de l'insuffisance rénale, de la maladie vasculaire périphérique, de l'hypertrophie ventriculaire gauche, d'un dysfonctionnement cognitif, de l'ictus, de la céphalée et de l'insuffisance cardiaque chronique, en particulier, de l'hypertension.
